# EUROPEAN PATENT APPLICATION

(11) **EP 2 626 109 A1**
(43) Date of publication of application: **14.08.2013**
(21) Application number: 12154380.5
(22) Date of filing: 08.02.2012
(51) Int. Cl.: A61N 1/05

(54) **A probe system for brain applications**

(71) Applicant: Sapiens Steering Brain Stimulation B.V., 5656 AE Eindhoven (NL)
(72) Inventor: Decré, Michel Marcel Jose, 5654 NC Eindhoven (NL); Tol, Jeroen Jacob Arnold, 5645 JG Eindhoven (NL); Martens, Hubert Cécile François, 5611 ND Eindhoven (NL)
(74) Representative: Rupprecht, Kay

(57) **Abstract**

The present inventions relates to a probe system (100) for brain applications, especially for neurostimulation and/or neurorecording, comprising at least one lead (300) for brain applications with a first number of stimulation and/or recording electrodes (132), the lead (300) being connectable to a low-count connector element (11) or having a low-count connector element (11), whereby the low-count connector element (11) has a second number of connector contacts, whereby the second number of connector contacts is lower than the first number of the electrodes (132), whereby the low-count connector element (11) is configured such that the low-count connector element (11) can be directly and/or indirectly connected to at least one pulse generator, whereby the probe system (10) further comprises a power-management unit (40), a controller unit (50), a communication unit (60) and a switching unit (70). Furthermore, the present invention relates to a deep brain stimulation system, a low-count connector element (11) and to a method of manufacturing a probe system (10) for a deep brain stimulation system.

## Description

The present invention relates to a probe system for brain applications and to a deep brain stimulation system and to a method of manufacturing a probe system for deep brain stimulation.

Implantable neurostimulation devices have been used for the past 10 years to treat acute or chronic neurological conditions. Deep brain stimulation (DBS), the mild electrical stimulation of sub-cortical structures, belongs to this category of implantable devices, and has been shown to be therapeutically effective for Parkinson's disease, Dystonia, and Tremor. New applications of DBS in the domain of psychiatric disorders (obsessive compulsive disorder, depression) are being researched and show promising results. In existing systems, the 1.27 mm-diameter, 10-50 cm-long leads carry 4 annular electrodes at the distal end, that are connected to the Implantable Pulse Generator (IPG) using a 3.8 mm-diameter, 4 screw-contacts connector, by means of 2.8 mm-diameter extension cables. The proximal end of the lead has four concentric contacts that fit into the 4-contacts connector of the extension cable, thereby electrically connecting each electrode to the outputs of the IPG through a so-called "header".

Future systems will need more, smaller electrodes, in order to better control the delivery of electrical stimulation, because current stimulation causes mild to severe side-effects in about 30% of the patients (see e.g. Burdick, et. al., Relationship between higher rates of adverse events in deep brain stimulation using standardized prospective recording and patient outcomes, in: Neurosurg Focus 29 (2): E4, 2010; and Temel, et. al., Behavioural changes after bilateral subthalamic stimulation in advanced Parkinson disease: A systematic review, in: Parkinsonism and Related Disorders 12 (2006) 265-272). A larger number of electrodes means a larger number of contacts to the connector, which in turn calls for different connector technologies, because it cannot be expected from the neurosurgeon to tighten more than 10 individual screws for the more than 10 contacts. Also, the contact sizes need to be smaller, certainly in the case of cranial implants.

One drawback of existing neurostimulation is that existing neurostimulation devices can only address a small number of electrodes, in particular 16 electrodes as a maximum, due to practical limitations on the number of connections that can be connected intra-operatively. Furthermore, due to the fact that existing implantable pulse generators should be modified as little as possible for cost reasons and for back-compatibility considerations, any improvements should leave the design of the implantable pulse generator untouched.

US 6,038,480 proposes implantable controller means that are integrated in the lead at a site adjacent to the tissue to be stimulated with a main cable having at least one power conductor adapted to extend to a site adjacent said tissue, wherein the number of set power conductors is fewer than the number of said electrodes and where the cable connects to the implantable controller means. The system further comprises a source of data and a data conductor. The implantable controller of US 6,038,480 is further configured to establish an anode and cathode relationship between pairs of nonadjacent electrodes.

Although reducing the number of connections to be realized by the surgeon while increasing the number of addressable electrodes, the system according to US 6,038,480 has the disadvantage of increasing the size of the lead and may require significant changes to the standard procedure currently used to implant leads as well as the manufacturing procedures, as e.g. outlined in US 7,286,878 B2.

US 7,286,878 B2 and EP 1 446 189 B1 claim to solve this technical problem by an extension unit that is coupled between the implantable pulse generator and the implantable electrode array and is configured to electrically connect the output sources to a portion of the electrodes.

While having the advantage of not requiring to change the surgery for lead implantation and manufacturing procedures of leads, the systems according to US 7,286,878 B2 and EP 1 446 189 B1 do not at all address the problem of many connections that have to be made intra-operatively, a fact further stressed as an extension unit is also implanted between the electrode array and the pulse generator. Such a solution requires an additional device to be implanted and becomes prohibitive when the number of contacts exceeds 10 or more.

The system according to US 7,286,878 B2 has the further disadvantage that each switch is being connected between one output source and at least a portion of the electrodes to simultaneously trigger a plurality of electrodes. This significantly restricts the connection options, which is not desired.

It is therefore an object of the present invention, to provide a probe system for brain applications, which is able to stimulate with higher electrode-count probes for more accurate delivery of therapeutic stimulation to a neural tissue.

The above object is solved according to the present invention by probe system for brain applications with the features of claim 1. Accordingly, a probe system for brain applications comprises at least one lead for brain applications with a first number of stimulation and/or recording electrodes, the lead being connectable to a low-count connector element or having a low-count connector element, whereby the low-count connector element has a second number of connector contacts, whereby the second number of connector contacts is lower than the first number of the electrodes, whereby the low-count connector element is configured such that the low-count connector element can be directly and/or indirectly connected to at least one pulse generator, whereby the probe system further comprises a power-management unit, a controller unit, a communication unit and a switching unit.

The probe system for brain applications may be a probe system especially for neurostimulation and/or neurorecording applications, whereby the probe system is preferably a probe system of a deep brain stimulation system. Moreover, the probe system may be completely implantable and embodied as a long-term implant.

The stimulation and/or recording electrodes may be arranged on the outer body surface of the probe, especially at the distal end of the probe.

While providing significant functionality like selecting electrodes for steering, supporting recording, and other sensing functions, it is preferable that a probe or lead having a large number of electrodes would not carry the power supply (e.g. a primary or rechargeable battery), nor the pulse generation function. These can more conveniently be present in a separate IPG. One advantage is that the components that are most likely to be replaced can be replaced easily. Another advantage is that an existing IPG needs only little modification to be able to communicate with and control the probe system.

The present invention is especially based on the recognition that typical stimulation power supplied by a standard implantable pulse generator unit (IPG) is 100 µW, whereas only 10 µW or much less is needed to power the circuitry (with e.g. the components Power Management Unit (P), Switching Unit (S), Communication Unit (C), Control unit (u)). Thus by adding electronics to the connector, a standard non-modified IPG can be used for both delivery of therapeutic stimulation pulses and power (to be scavenged from pulses). Indeed, in the case of a non-modified IPG, said IPG has no means to appropriately communicate instructions to the active lead, e.g. regarding which electrodes have to be grouped or addressed with which stimulation pulses.

In a particularly interesting option, a single external control unit communicates with both the IPG and the active high-resolution probe, without the user having to use separate control units.

An additional advantage is thus that several IPG types from different vendors or manufacturers can easily be connected and communicate with such a probe or lead.

Moreover, it is possible that the low-count connector element comprises a probe connector enclosure, whereby a low-count connector, the power-management unit, the controller unit, the communication unit and the switching unit is integrated into the probe connector enclosure.

Additionally, the probe system may comprise a recording unit, whereby preferably the recording unit is integrated into the probe connector enclosure. It is possible to use the recording unit to record data of interest, e.g. parameters of a deep brain stimulation treatment. The recording unit may be preferably configured such that any signals recorded by one or more of the electrodes may be recorded. For instance, these signals may comprise electrical signals of the brain tissue adjacent to the lead and/or of the brain tissue be stimulated by the probe system.

The probe connector enclosure may be a connector box having a bottom wall or top wall and side walls with a high-count feed-through at the bottom wall or top wall and a low-count feed-through at least one side wall, whereby in the inside of the connector box the power-management unit, the controller unit, the communication unit, the switching unit and/or the recording unit is arranged, and whereby a low-count connector is arranged and connected to the low-count feed-through. The bottom wall or the top wall may be the wall or surface of the connector box having the largest dimensions when compared with all other outer surfaces of the connector box. Generally, it is also possible that the high-count feed-through is arranged in at least one side wall of the connector box.

Furthermore, it is possible that the probe system is configured such that the probe can be used for delivery of stimulation to a neuronal tissue, e.g. brain tissue by connecting it to a conventional pulse-generator unit that supplies stimulation pulses, whereby the pulse-generator unit is preferably an implantable pulse-generator unit.

Additionally, it is possible that the communication unit is configured such that the communication unit is capable of a communicating wirelessly or over an electrical line for sending data and status information and/or for receiving programming commands. The communication may be established by using optical, magnetic, mechanical (for instance by using vibration or ultrasound) or other suitable communication means.

Preferably, the controller unit may be configured such that the controller unit is capable to configure the switching unit and/or capable to receive and to transmit data with the communication unit.

Further preferably, it is possible that the switching unit is configured such that the switching unit is configurable to relay incoming stimulation pulses from a connected and/or connectable pulse-generator to any arbitrary combination of electrodes on the distal end of the probe.

Advantageously, the power management unit may be configured such that the power management unit is capable of extracting electrical power wirelessly or from incoming electrical lines to power the electronic circuitry integrated into the probe system.

Further advantageously, it is possible that the power-management unit is configured such that the power-management unit is a power-scavenging unit being able to extract power from an electrical line carrying stimulation pulses from the connected and/or connectable pulse-generator.

Additionally, the present invention relates to a low-count connector element with the features of claim 10. Accordingly, a low-count connector element comprises the features of the low-count connector element according to any of claims 1 to 9.

In particular, the low-count connector element comprises a probe connector enclosure, whereby a low-count connector, the power-management unit, the controller unit, the communication unit and the switching unit is integrated into the probe connector enclosure. Especially, the power-management unit, the controller unit, the communication unit and the switching unit may be embodied as described above.

Moreover, the present invention relates to a deep brain stimulation system with the features of claim 11. Accordingly, a deep brain stimulation system comprises at least one probe system according to one of claims 1 to 9 and/or comprises at least one low-count connector element according to claim 10. In one possible embodiment deep brain stimulation system may comprise the probe system only, thus the deep brain stimulation system being a probe system according to any of claims 1 to 9.

It is possible, that the deep brain stimulation system further comprises at least one external control unit configured such that the external control unit can communicate directly and/or indirectly with a probe of probe system, whereby the deep brain stimulation system further comprises at least one pulse-generator, preferably a pulse-generator of an implantable pulse-generator unit, whereby the external control unit and the pulse-generator are configured such that the external control unit and the pulse-generator can communicate directly and/or indirectly.

Advantageously, such an additional, external unit can be used to control the active high-resolution probe, in order to transmit instructions of which electrodes have to be addressed by an implantable pulse generator unit.

Furthermore, it is possible that the probe is a high-resolution active probe, whereby the deep brain stimulation system further comprises an implantable pulse generator unit and an external control unit.

Additionally, the present invention relates to a method of manufacturing a probe system for a deep brain stimulation system with the features of claim 14. Accordingly, a method of manufacturing a probe system for a deep brain stimulation system is disclosed, whereby the probe system is a probe system according to any of claims 1 to 9 and/or whereby the deep brain stimulation system is a deep brain stimulation system according to any of claims 11 to 13.

Advantageously, it is possible that as at least one component of the probe system a probe connector enclosure of a low-count connector element is manufactured, whereby the probe connector enclosure is preferably embodied as a connector box, the probe connector enclosure having a bottom wall or top wall and side walls, whereby the probe connector enclosure has a high-count feed-through at the bottom wall or top wall and a low-count feed-through at least one side wall, whereby in the inside of the probe connector enclosure electronic components of the probe system are arranged, whereby the electronic components comprise preferably at least one of the power-management unit, the controller unit, the communication unit, the switching unit and/or the recording unit, whereby a low-count connector, preferably a low-count connector block, is arranged and connected to the low-count feed-through at the side wall of the connector enclosure, whereby the electronic components are connected directly and/or indirectly to the electrodes of the probe and whereby a header element is attached to the lead connector enclosure for closing the probe connector enclosure to form the low-count connector element.

Further details and advantages of the present invention shall be described hereinafter with respect to the drawings:
- Fig. 1:: a schematical drawing of a neurostimulation system for deep brain stimulation (DBS);
- Fig. 2:: a further schematical drawing of a probe neurostimulation system for deep brain stimulation (DBS) and its components;
- Fig. 3:: a schematical drawing of a probe system according to the present invention;
- Fig. 4:: a more detailed schematical drawing of the probe system shown in Fig. 3;
- Fig. 5:: a further schematical drawing of the probe system shown in Fig. 3 and 4; and
- Fig. 6A-F:: the steps of the manufacturing steps of the probe system shown in Fig. 3 to 5.

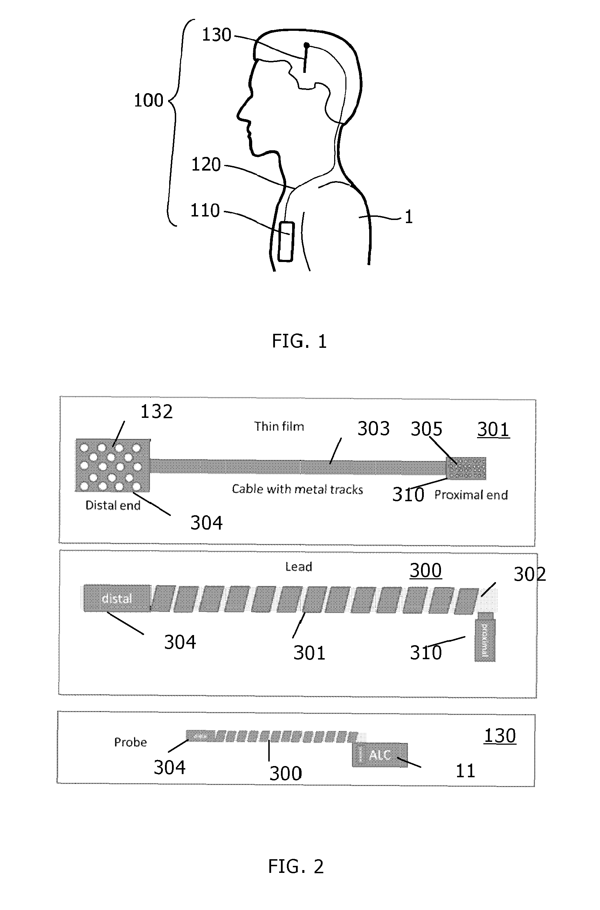

A possible embodiment of a neurostimulation system 100 for deep brain stimulation (DBS) is shown in Figure 1. The neurostimulation system 100 comprises at least a controller 110 that may be surgically implanted in the chest region of a patient 1, typically below the clavicle or in the abdominal region of a patient 1. The controller 110 can be adapted to supply the necessary voltage pulses. The typical DBS system 100 may further include an extension wire 120 connected to the controller 110 and running subcutaneously to the skull, preferably along the neck, where it terminates in a connector. A DBS lead arrangement 130 may be implanted in the brain tissue, e.g. through a burr-hole in the skull.

Figure 2 further illustrates a typical architecture for a Deep Brain Stimulation probe 130 that comprises a DBS lead 300 and an Advanced Lead Connector (ALC) element 11 comprising electronic means to address electrodes 132 on the distal end 304 of the DBS lead 300. The lead 300 comprises a carrier 302 for a thin film 301, said carrier 302 providing the mechanical configuration of the DBS lead 300 and the thin film 301. The thin film 301 may include at least one electrically conductive layer, preferably made of a biocompatible material. The thin film 301 is assembled to the carrier 302 and further processed to constitute the lead element 300. The thin film 301 for a lead is preferably formed by a thin film product having a distal end 304, a cable 303 with metal tracks and a proximal end 310. The proximal end 310 of the thin film 301 on the lead 300 is electrically connected to the ALC element 11. The ALC element 11 comprises the switch matrix of the DBS steering electronics. The distal end 304 comprises the electrodes 132 for the brain stimulation. The proximal end 310 comprises the interconnect contacts 305 for each metal line in the cable 303. The cable 303 comprises of metal lines (not shown) to connect each distal electrodes 132 to a designated proximal contact 305.

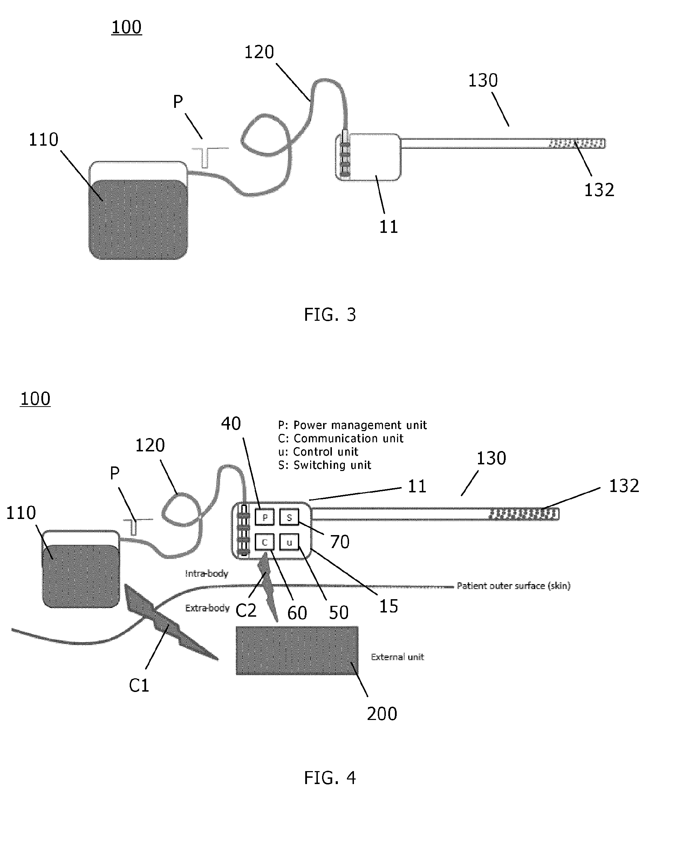

Figure 3 shows schematically and in greater detail an embodiment of a system 100 for brain applications, here for neurostimulation and/or neurorecording as a deep brain stimulation system 100 as shown in Figures 1 and 2. The probe system 100 comprises at least one probe 130 for brain applications with stimulation and/or recording electrodes 132, whereby e.g. 64 electrodes 132 can be provided on outer body surface at the distal end of the probe 130. By means of the extension wire 120 pulses P supplied by controller 110 can be transmitted to the ALC 11. The controller 110 can be an implantable pulse generator (IPG) 110.

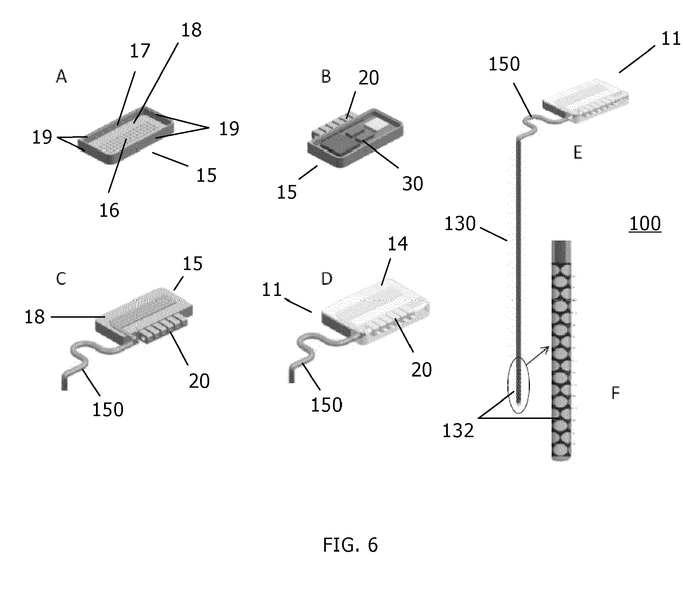

The lead 300 of the probe 130 is connected to a low-count connector element 11 (see e.g. Figure 6), whereby the low-count connector element 11 has a low-count connector 20 with a second number of connector contacts, whereby the second number of connector contacts is lower than the first number of the electrodes 132, whereby the low-count connector element 11 is configured such that the low-count connector 20 can be directly and/or indirectly connected to at least one pulse generator of an implantable pulse generator unit (IPG) 110. As can be derived from Figure 6, the low-count connector element 11 also comprises the electronic components 30 of the probe system 100 according to embodiment of the present invention described hereinafter.

The probe system 100 is configured such that the lead 300 can be used for delivery of stimulation to a neuronal tissue, e.g. brain tissue by connecting it to a conventional pulse-generator unit that supplies stimulation pulses, whereby the pulse-generator unit is preferably an implantable pulse-generator unit 110. Generally, the probe system 100 according to the present invention can easily be connected and communicate with several IPG types from different vendors or manufacturers, e.g. by means of the connecting extension wire 120, which is in particular transmitting stimulation pulses P generated by the pulse generator.

As further shown in detail in Figure 4, the low-count connector element 11 further comprises as electronic components 30 a power-management unit 40, a controller unit 50, a communication unit 60 and a switching unit 70. These electronic components 30 are integrated into the probe connector enclosure 15.

The controller unit 50 can be a microprocessor unit or a combinational logic unit or a sequential logic unit (e.g. a state machine), which is however not mandatory. Further embodiments of the controller unit 50 are possible.

Additionally, the low-count connector element 11 may comprise a recording unit (not shown), whereby preferably the recording unit is integrated into the probe connector enclosure 15. The probe connector enclosure 15 is a connector box typically having a bottom 18 or top wall (not shown) and side walls 19 with a high-count feed-through 16 at the bottom 18 or top wall, and a low-count feed-through 17 on the side wall 19, whereby in the inside of the connector box the power-management unit 40, the controller unit 50, the communication unit 60, the switching unit 70 and the recording unit is arranged, and whereby a low-count connector 20 is arranged and connected to the low-count feed-through 17 (see also Figure 6).

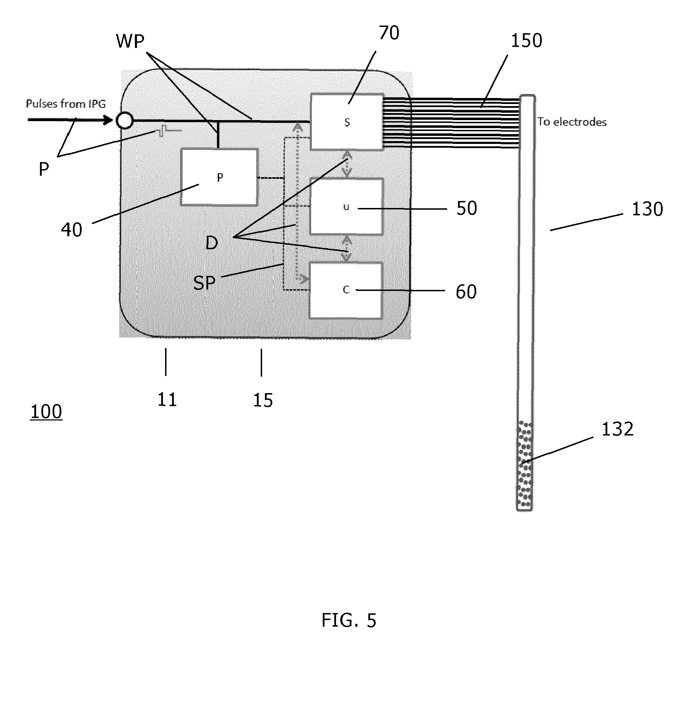

The communication unit 60 is configured such that the communication unit 60 is capable of a communicating wirelessly or over an electrical line for sending data and status information and/or for receiving programming commands. The controller unit 50, which can be e.g. a microprocessor unit, is configured such that the controller unit 50 is capable to configure the switching unit 70 and capable to receive and to transmit data with the communication unit 60, e.g. via wiring of data signals D (see also Figure 5). The switching unit 70 is configured such that the switching unit 70 is configurable to relay incoming stimulation pulses from a connected and/or connectable pulse-generator to any arbitrary combination of electrodes 132 on the distal end of the lead 300 of the probe 130.

Furthermore, the power management unit 40 is configured such that the power management unit 40 is capable of extracting electrical power wirelessly or from incoming electrical lines to power the electronic circuitry integrated into the probe system 100. The power-management unit 40 can be configured such that the power-management unit 40 is a power-scavenging unit being able to extract power from an electrical line carrying stimulation pulses from the connected and/or connectable pulse-generator (see also Figure 5).

The deep brain stimulation system 100 further comprises at least one external control unit 200, which is arranged outside of the body of the patient (extra-body) and which is configured such that the external control unit 200 can communicate with the communication unit 60 of the connector box 11 of the probe system 100. This is indicated in Figure 4 by the communication stream C2.

As shown Figure 4, the probe system 100 and in particular the low-count connector element 11 is also implanted into the patient likewise the probe 130 and the implantable pulse generator unit 110 (intra-body arrangement).

The external control unit 200 and the pulse-generator of the implantable pulse generator unit 110 are configured such that the external control unit 200 and the pulse-generator can communicate directly through the skin of the patient wirelessly, which is indicated by the communication stream C1.

The external control unit 200 can supply communication including the possibility of a data exchange, control and may also act as a wireless power supply, e.g. to recharge power storage means like rechargeable batteries of the implantable pulse generator unit 110.

As shown in Figure 5, the probe system 100 has a so-called wiring of pulses WP, a so-called wiring of scavenged power SP and a so-called wiring of data signals D. The switching unit 70 is connected to a connection 150, which is the connection to the probe 130 and its electrodes 132.

Figures 6A-F show several steps of the method of manufacturing a probe system 100 for a deep brain stimulation system 100 and also some details of the probe system 100 for a deep brain stimulation system.

As shown in Figure 6A, a probe connector enclosure 15 is used, whereby the probe connector enclosure 15 is embodied as a connector box. The probe connector enclosure 15 has a bottom 18 or top wall and side walls 19, whereby the probe connector enclosure 15 has a high-count feed-through 16 at the bottom 18 and a low-count feed-through 17 at least one side wall 19.

Furthermore and as shown in Figure 6B, in the inside of the probe connector enclosure 15 electronic components 30 of the probe system 100 are arranged, whereby the electronic components 30 comprise preferably at least one of the power-management unit 40, the controller unit 50, the communication unit 60, the switching unit 70 and/or the recording unit. According to the embodiment of the probe system 10 described above and shown in Figures 6A-F the power-management unit 40, the controller unit 50, the communication unit 60, the switching unit 70 and the recording unit are arranged inside of the probe connector enclosure 15 of the low-count connector element 11.

Additionally, a low-count connector 20, preferably a low-count connector block, is arranged and connected to the low-count feed-through 17 at the side wall 19 of the connector enclosure 15.

In the next step, which is shown in Figure 6C, the electronic components are connected indirectly to the electrodes 132 of the probe 131 via connection 150 (see also Figure 5).

As further shown in Figure 6D, a header element 14 is attached to the probe connector enclosure 15 for closing the probe connector enclosure 15.

The manufactured probe system 100, in particular the low-count connector element 11 is shown in Figure 6E, whereby Figure 6F shows an enlarged and detailed view of the distal end of the probe 130 with the electrodes 132.

## Claims

1. A probe system (100) for brain applications, especially for neurostimulation and/or neurorecording, comprising at least one lead (300) for brain applications with a first number of stimulation and/or recording electrodes (132), the lead (300) being connectable to a low-count connector element (11) or having a low-count connector element (11), whereby the low-count connector element (11) has a second number of connector contacts, whereby the second number of connector contacts is lower than the first number of the electrodes (132), whereby the low-count connector element (11) is configured such that the low-count connector element (11) can be directly and/or indirectly connected to at least one pulse generator, whereby the probe system (10) further comprises a power-management unit (40), a controller unit (50), a communication unit (60) and a switching unit (70).

2. The probe system (100) according to claim 1,
**characterized in that**
the low-count connector element (11) comprises a probe connector enclosure (15), whereby a low-count connector (20), the power-management unit (40), the controller unit (50), the communication unit (60) and the switching unit (70) is integrated into the probe connector enclosure (15).

3. The probe system (100) according to claim 1 or 2, **characterized in that**
the probe system (100) comprises a recording unit, whereby preferably the recording unit is integrated into the probe connector enclosure (15).

4. The probe system (100) according to any of claims 2 or 3, **characterized in that**
the probe connector enclosure (15) is a connector box having a bottom wall (18) or top wall and side walls (19) with a high-count feed-through (16) at the bottom wall (18) or top wall and a low-count feed-through (17) at least one side wall (19), whereby in the inside of the connector box the power-management unit (40), the controller unit (50), the communication unit (60), the switching unit (70) and/or the recording unit is arranged, and whereby a low-count connector (20) is arranged and connected to the low-count feed-through (17).

5. The probe system (100) according to any of the preceding claims, **characterized in that**
the probe system (100) is configured such that the lead (300) can be used for delivery of stimulation to a neuronal tissue, e.g. brain tissue by connecting it to a conventional pulse-generator unit that supplies stimulation pulses, whereby the pulse-generator unit is preferably an implantable pulse-generator unit (110).

6. The probe system (100) according to any of the preceding claims, **characterized in that**
the communication unit (60) is configured such that the communication unit (60) is capable of a communicating wirelessly or over an electrical line for sending data and status information and/or for receiving programming commands.

7. The probe system (100) according to any of the preceding claims, **characterized in that**
the controller unit (50) is configured such that the controller unit (50) is capable to configure the switching unit (70) and/or capable to receive and to transmit data with the communication unit (60).

8. The probe system (100) according to any of the preceding claims, **characterized in that**
the switching unit (70) is configured such that the switching unit (70) is configurable to relay incoming stimulation pulses from a connected and/or connectable pulse-generator to any arbitrary combination of electrodes (132) on the distal end of the lead (300).

9. The probe system (100) according to any of the preceding claims, **characterized in that**
the power management unit (40) is configured such that the power management unit (40) is capable of extracting electrical power wirelessly or from incoming electrical lines to power the electronic circuitry integrated into the probe system (100) and/or the power-management unit is (40) configured such that the power-management unit (40) is a power-scavenging unit being able to extract power from an electrical line carrying stimulation pulses from the connected and/or connectable pulse-generator.

10. A low-count connector element (11) comprising the features of the low-count connector element (11) according to any of claims 1 to 9.

11. A deep brain stimulation system comprising at least one probe system (100) according to any of claims 1 to 9 and/or comprising at least one low-count connector element (11) according to claim 10.

12. The deep brain stimulation system according to claim 11, **characterized in that**
the deep brain stimulation system further comprises at least one external control unit (200) configured such that the external control unit (200) can communicate directly and/or indirectly with a probe (130) of probe system (100), whereby the deep brain stimulation system further comprises at least one pulse-generator, preferably a pulse-generator of an implantable pulse-generator unit (110), whereby the external control unit (200) and the pulse-generator are configured such that the external control unit (200) and the pulse-generator can communicate directly and/or indirectly.

13. The deep brain stimulation system according to claim 11 or 12, **characterized in that**
the probe (130) is a high-resolution active probe, whereby the deep brain stimulation system (100) further comprises an implantable pulse generator unit (110) and an external control unit (200).

14. A method of manufacturing a probe system (100) for a deep brain stimulation system, whereby the probe system (100) is a probe system (100) according to any of claims 1 to 10 and/or whereby the deep brain stimulation system is a deep brain stimulation system according to any of claims 11 to 13.

15. The of manufacturing a probe system (100) for a deep brain stimulation system according to claim 14,
**characterized in that**
as at least one component of the probe system (100) a probe connector enclosure (15) of a low-count connector element (11) is manufactured, whereby the probe connector enclosure (15) is preferably embodied as a connector box, the probe connector enclosure (15) having a bottom wall (18) or top wall and side walls (19), whereby the probe connector enclosure (15) has a high-count feed-through (16) at the bottom wall (18) or top wall and a low-count feed-through (17) at least one side wall (19), whereby in the inside of the probe connector enclosure (15) electronic components (30) of the probe system (100) are arranged, whereby the electronic components (30) comprise preferably at least one of the power-management unit (40), the controller unit (50), the communication unit (60), the switching unit (70) and/or the recording unit, whereby a low-count connector (20), preferably a low-count connector block, is arranged and connected to the low-count feed-through (17) at the side wall (19) of the connector enclosure (15), whereby the electronic components (30) are connected directly and/or indirectly to the electrodes (15) of the lead (300) and whereby a header element (14) is attached to the probe connector enclosure (15) for closing the probe connector enclosure (15) to form the low-count connector element (11).
